# EUROPEAN PATENT APPLICATION

(11) **EP 0 623 359 A2**
(43) Date of publication of application: **09.11.1994**
(21) Application number: 94302972.8
(22) Date of filing: 25.04.1994
(51) Int. Cl.: A61M 5/32, A61B 5/14

(54) **Safety needle sheaths**

(30) Priority: 06.05.1993 US 57778
(71) Applicant: SMITHS INDUSTRIES MEDICAL SYSTEMS INC., Keene, New Hampshire 03431-0724 (US)
(72) Inventor: Hollister, William Hermann, East Sullivan, New Hampshire 03445 (US)
(74) Representative: Flint, Jonathan McNeill

(57) **Abstract**

A safety device for an evacuted blood-sampling tube holder 28 or a syringe 38 has a housing 8 flexibly connected to a base 4 and pivotable to a position where it aligns and locks with a needle 16 projecting from one end of the base. A cap 26 projects from the other end of the base to encircle the tube holder or syringe barrel. The cap 26 has notches 28 that engage fingers 30 projecting from the holder or barrel so that the device can be uncoupled after use.

## Description

This invention relates to safety devices of the kind including a base having a first end for connection with a needle and a housing flexibly connected to the base and pivotable to a position in substantial alignment along the longitudinal axis of the needle for enveloping the needle.

In US4982842 there is disclosed a safety needle container, for use with a syringe, which protects a user from being accidentally pricked by the sharp end of a needle. US5139489 describes a safety sheath mated to a holder for an evacuated tube, such as a vacutainer. In US5154285 there is described a device with a safety needle sheath rotatably mounted about the neck of a tube holder. The disclosures of the above patents are hereby incorporated to this application by reference.

US 5139489 also discloses a variant in which the safety needle sheath is removable from the tube holder. However, this variant (shown in Figure 4 of US 5139489) requires that a specially designed safety needle sheath adapter be threadedly mated to a tube holder; and that after use, the housing be removed from the tube holder. This variant was found to be impractical because of its dimensional requirements.

It is an object of the present invention to provide an improved safety device for protecting a needle and which can be releasably coupled to a fluid receptacle.

According to the present invention there is provided a safety device of the above-specified kind, characterised in that the device has a cap extending from a second end of the base for releasably coupling the base to a fluid receptacle.

The fluid receptacle may comprise a holder within which a fluid container is insertable. The needle preferably has a first end for insertion into a patient and a second end extending into the holder to be in fluid communication with the fluid container. The cap preferably extends from the second end of the base to a length at least as long as the second end of the needle.

Alternatively, the fluid receptacle may be a syringe.

The cap may be coupled to the receptacle via a first latch on the cap and a corresponding second latch on the receptacle. The housing may include a member for preventing relative movement between the housing and the needle once the housing has been pivoted to the alignment position. The retaining member may include at least one hook integral with the housing for securely retaining the needle within the housing to prevent relative movement between the needle and the housing. The housing may be rotatably mounted about the base. The safety device may include fluid-absorbing material mounted on the housing and configured to contact the needle to absorb fluid thereon before the housing is pivoted fully to the alignment position.

A safety device in accordance with the present invention, will now be described, by way of example, with the reference to the accompanying drawings, in which:
- Figure 1: is a partly cutaway cross-sectional view of the device;
- Figure 2: is a partly cutaway cross-sectional view of a second embodiment of the present invention; and
- Figure 3: is a partly cutaway cross-sectional view showing modifications to the embodiment in Figure 1.

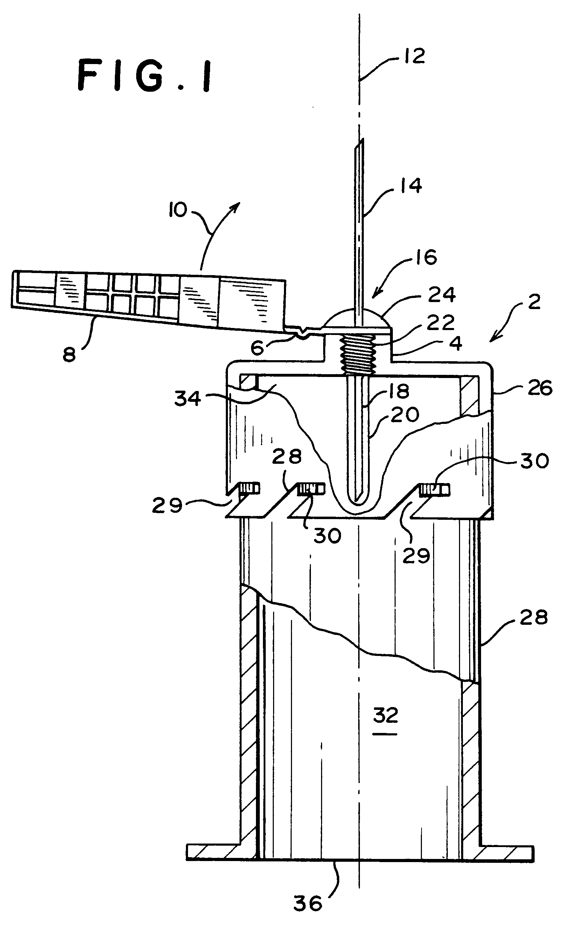

With reference to Figure 1, the safety device 2 has a base 4 connected via a flexible connection or living hinge 6 to a sheath or housing 8. The housing 8 is pivotable along the direction indicated by the arrow 10 to be in substantial alignment along the longitudinal axis 12 of a cannula 14. The cannula 14 is a first end of a double-ended needle 16 the other end of which is a second cannula 18 enclosed by a rubber sheath 20. The double-ended needle 16 is threadedly mated to the base 4 via a threaded portion 22 extending from its hub 24.

Extending from the lower portion of the base 4 is a skirt or cap 26 of circular section having an diameter equal to the outer diameter of a tube holder 28. The cap 26 has at its lower end a number of latches in the form of notches 29 shaped to mate and lock with a corresponding number of latches in the form of fingers 30 extending radially from the tube holder 28. The tube holder 28 provides a fluid receptacle into the interior 32 of which a conventional fluid container, such as a vacuum tube or vacutainer, can be inserted to be in fluid communication with the double-ended needle 16. As shown, unlike conventional vacutainer holders, the tube holder 28 can be opened at both ends. Of course, it should be appreciated that the end 34 of the tube holder 28 may have a smaller opening than the end 36, inasmuch as the size of the opening at the end 34 is not that significant, so long as the rubber sheath 20 can be fitted thereat. In any event, once the cap 26 is fitted over the tube holder 28 and is turned clockwise approximately one quarter turn so that the notches 29 are lockingly secured to the fingers 30, the device is ready for use. The length of the cap 26 along the axis 12 extending from the base 4 is sufficiently long (that is, at least as long as the second cannula 18) such that the tip of the cannula 18 would not be exposed if the cap 26 were removed from the tube holder 28.

In use, the cannula 14 is inserted into a blood vessel and an evacuted tube is pushed into the holder 28 towards the cannula 18. The evacuated tube has a self-sealing, piercable bung at one end, which is pierced by the cannula 18 so that the reduced pressure in the tube is communicated to the needle 16. This causes blood to be sucked along the needle 16 into the tube in the usual way.

After use, that is, after the cannula 14 has been withdrawn from the patient, the sheath 8 is pivoted along the arrow 10 to a position in substantial alignment along the longitudinal axis 12 thereby to envelop the cannula 14. A locking mechanism, such as a number of hooks, may be formed in the sheath 8 to snap onto the cannula 14 and lock it within the sheath. Thereafter, the device 2 is removed from the tube holder 28, by twisting the cap 26 in a counter-clockwise fashion so that it no longer is secured by the fingers 30. The safety device 2 can then be disposed of safely. The tube holder 28, because it is of a sturdy polymer material, can be reused after it has been sterilized, thereby saving waste material.

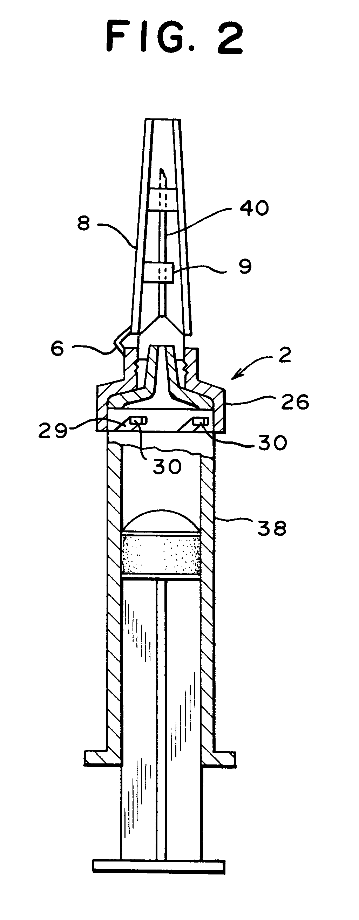

Figure 2 illustrates a modification of the safety device 2 shown in the Figure 1 in which the fluid receptacle is a syringe 38. As shown, the syringe 38, fitted with a needle assembly 40, is mated to a safety device 2 via its cap 26. A number of fingers 30 extending from the syringe 38 secure the cap 26 to the syringe by means of notches 29 in the cap. The operation of the syringe of Figure 2 is similar to that discussed with reference to the tube holder of Figure 1. Thus, after use, the safety device 2 is twisted off the syringe 38 and disposed of. The syringe 38 is likewise disposed of.

In the Figures 1 and 2 embodiments, the tube holder 28 and syringe 38 are specially manufactured to include the projecting fingers 30.

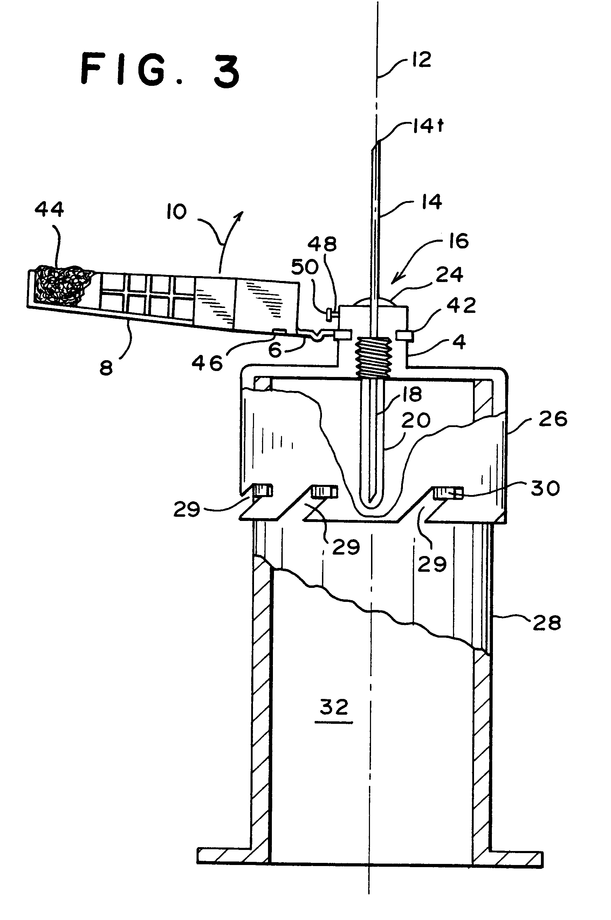

Variants of the invention are shown in Figure 3. It should be appreciated that these variants are equally applicable to the syringe embodiment shown in Figure 2. Components in Figure 3 that are the same as those shown in Figure 1 are labelled the same.

The Figure 3 embodiment illustrates the rotatable mounting of the sheath 8 to the base 4 by means of a non-enclosed ring 42, such as that disclosed in US 5154285. Accordingly, the sheath 8 is rotatable about the base 4 to enable the user to rotate the sheath so that it does not provide an obstruction during use and so that the tip 14t of the cannula 14 can be clearly viewed.

A second variant of the Figure 1 embodiment, as shown in Figure 3, is the inclusion of a fluid-absorbent material 44 mounted on the end portion of the sheath 8. Such fluid-absorbent material 44 may include, for example, foam, paper, sponge or other materials that can readily absorb fluid, such as blood that may be formed at the tip of cannula 14, after it is withdrawn from a patient. The fluid-absorbent material 44 is shaped and positioned such that it contacts the cannula 14 before any hook 9. Thus, as the sheath 8 is pivoted toward the axis 12, the fluid-absorbent material 44 absorbs any fluid formed at the tip 14t of the cannula 14 before the hook 9 contacts the cannula. Thus, even if the hook 9 were to impart a motion to the cannula 14 to cause it to shake, there would be no danger of any fluid being flicked into the environment, since such fluid would have been absorbed by the material 44 before the cannula contacts the hook. The nature of the material 44 is such that it readily yields to the cannula 14 as it contacts the cannula.

Yet another variant of the present invention uses a cooperating locking mechanism between the sheath 8 and the base 4 to prevent further relative movement between the sheath 8 and the base 4 once the sheath has been pivoted to a position substantially in alignment along the axis 12. For this variant, instead of a hook in the sheath 8, an opening 46 is provided at the lower portion of the sheath and an extension 48 projects from the base 4 (assuming that the sheath is no longer rotatable about the base 4). The extension 48 has an enlarged, mushroom-shaped front end 50 with the tip and base portion of the front end being respectively configured to be smaller and larger than that of the opening 46. Thus, as the sheath 8 is pivoted toward the alignment position at the axis 12, the front end 50 penetrates through the opening 46. After the sheath 8 has been positioned in alignment with axis 12, the base portion of the front end 50 prevents the sheath from pivoting backwards, thereby preventing any further relative movement between the sheath and the base. Other types of locking mechanisms based on cooperation between means at the base and sheath are also envisioned. For example, a plurality of openings may be formed at the sheath to coact cooperatively with and lock onto corresponding tabs formed at the base.

## Claims

1. A safety device including a base (4) having a first end for connection with a needle (16) and a housing (8) flexibly connected to the base and pivotable to a position in substantial alignment along the longitudinal axis of the needle for enveloping the needle, characterised in that the device has a cap (26) extending from a second end of the base (4) for releasably coupling the base to a fluid receptacle (28, 38).

2. A safety device according to Claim 1, characterised in that the fluid receptacle comprises a holder (28) within which a fluid container is insertable.

3. A safety device according to Claim 2, characterised in that the needle (16) has a first end (14) for insertion to a patient and a second end (18) extending into the holder (28) to be in fluid communication with the fluid container.

4. A safety device according to Claim 3, characterised in that the cap (26) extends from the second end of the base (4) to a length at least as long as the second end (18) ofthe needle (16).

5. A safety device according to Claim 1, characterised in that the fluid receptacle is a syringe (38).

6. A safety device according to any one of the preceding claims, characterised in that the cap (26) is coupled to the receptacle (28, 38) via a first latch (29) on the cap and a corresponding second latch (30) on the receptacle (28, 38).

7. A safety device according to any one of the preceding claims, characterised in that the housing (8) includes a member (9, 46) for preventing relative movement between the housing (8) and the needle (16) once the housing has been pivoted to the alignment position.

8. A safety device according to Claim 7, characterised in that the retaining member includes at least one hook (9) integral with the housing (8) for securely retaining the needle (16) within the housing to prevent relative movement between the needle (16) and the housing (8).

9. A safety device according to any one of the preceding claims, characterised in that the housing (8) is rotatably mounted about the base (4).

10. A safety device according to any one of preceding claims, characterised in that the device includes fluid-absorbing material (44) mounted on the housing and configured to contact the needle (16) to absorb fluid thereon before the housing (8) is pivoted fully to the alignment position.
